# EUROPEAN PATENT SPECIFICATION

(11) **EP 2 478 855 B1**
(45) Date of publication and mention of the grant of the patent: **07.09.2016**
(21) Application number: 12151483.0
(22) Date of filing: 18.01.2012
(51) Int. Cl.: A61B 17/34, A61B 10/02

(54) **Treatment device**
Behandlungsvorrichtung
Dispositif de traitement

(30) Priority: 24.01.2011 KR 20110006977; 24.01.2011 KR 20110006978
(43) Date of publication of application: 25.07.2012
(73) Proprietor: Samsung Medison Co., Ltd., Nam-myun Hongchun-gun Kangwon do 250-875 (KR)
(72) Inventor: Hyoun, Dong Gyu, Gyeonggi-do (KR); Song, Young Seuk, Seoul (KR)
(74) Representative: Schmid, Wolfgang

(56) References cited:
- US-A- 6 146 329
- US-A1- 2005 090 742
- US-A1- 2010 298 712

## Description

### BACKGROUND

### 1. Field

The present disclosure relates to the field of treatment devices. The disclosure is particularly applicable to treatment devices for measuring movement of a needle to control insertion length of the needle, thereby improving stability of a biopsy or treatment.

### 2. Description of the Related Art

A needle biopsy is used to diagnose a target, such as in biological tissue or a human body, suspected to be a tumor and choosing a treatment method based on such diagnosis.

One type of needle biopsy is an image guided needle biopsy using ultrasonic waves, X-ray, magnetic resonance imaging (MRI) or computer tomography (CT), in which an invisible needle is inserted into a human body.

A needle guide for guiding the needle is provided in a probe. One conventional probe is an ultrasonic diagnostic device. During biopsy, the needle moves along the needle guide to sample a target.

The above-mentioned technology is merely a related art provided to assist in understanding of the prevent disclosure.

In an ultrasonic image, the needle, moving to the front of the probe, is not easily distinguished from tissue around the needle. If a needle insertion route is not properly displayed in an image projected on an imaging device, it may be difficult for a user to confirm the movement distance of the needle. As a result, the possibility of error resulting in medical malpractice may be increased.

A treatment device according to the preamble of claim 1 is known from US patent no. 6 146 329.

### SUMMARY

It is an aspect of the present disclosure to provide a treatment device that accurately measures a movement distance of a needle, thereby improving stability of biopsy. Additional aspects of the disclosure will be set forth in the description which follows, will be obvious from the description, or may be learned by practice of, or techniques described in the disclosure.

In accordance with one aspect of the present disclosure, a treatment device according to claim 1 is provided.

### BRIEF DESCRIPTION OF THE DRAWINGS

The aspects of the disclosure will become apparent and more readily appreciated from the following description of the embodiments, taken in conjunction with reference to the accompanying drawings of which:
FIG. 1 is a perspective view showing a main unit connected to a treatment device according to an embodiment of the present disclosure;
FIG. 2 is an exploded perspective view of the treatment device according to the embodiment of the present disclosure;
FIG. 3 is an assembled perspective view of the treatment device according to the embodiment of the present disclosure;
FIG. 4 is a perspective view, partially cutaway, showing the construction of a measurement unit according to an embodiment of the present disclosure;
FIG. 5 is a sectional view showing a state of a needle of the treatment device according to the embodiment of the present disclosure before being withdrawn from a needle guide;
FIG. 6 is a sectional view showing a state of a first indicator according to an embodiment of the present disclosure measured by a camera;
FIG. 7 is a sectional view showing symbols to calculate a length of the needle of the treatment device according to the embodiment of the present disclosure inserted into a target;
FIG. 8 is a block diagram of the treatment device according to the embodiment of the present disclosure;
FIG. 9 is a sectional view showing a state of a needle of a treatment device according to another embodiment of the present disclosure before being withdrawn from a needle guide;
FIG. 10 is a sectional view showing a state of a first indicator measured by a camera and a first optical sensor according to another embodiment of the present disclosure;
FIG. 11 is a sectional view showing a state of a needle of a treatment device according to another embodiment of the present disclosure before being withdrawn from a needle guide;
FIG. 12 is a sectional view showing a state of a second indicator measured by an optical sensor and a second optical sensor according to another embodiment of the present disclosure;
FIG. 13 is a sectional view showing a state of a needle of a treatment device according to another embodiment of the present disclosure, however, not forming part of the present invention, before being withdrawn from a needle guide;
FIG. 14 is a sectional view showing a state of a coil sensor according to another embodiment, however, not forming part of the present invention, passing through a magnetic field generation unit together with a needle;
FIG. 15 is a sectional view showing a state of a needle of a treatment device according to another embodiment of the present disclosure, however, not forming part of the present invention, before being withdrawn from a needle guide;
FIG. 16 is a sectional view showing a state of a coil sensor according to another embodiment, however, not forming part of the present invention, passing through a magnetic field generation unit together with a needle;

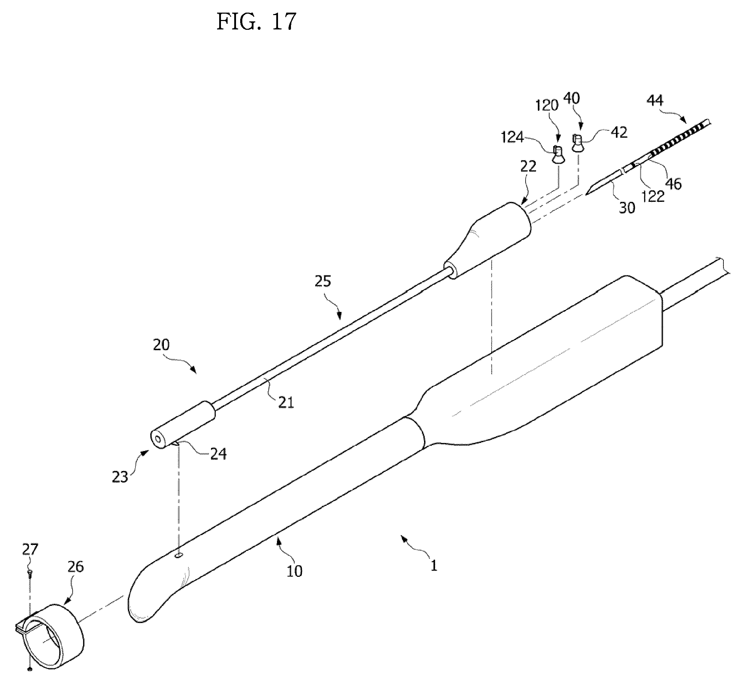

FIG. 17 is an exploded perspective view showing a treatment device according to another embodiment of the present disclosure;
FIG. 18 is an assembled perspective view of the treatment device according to the embodiment of the present disclosure;
FIG. 19 is a perspective view, partially cutaway, showing the constructions of an operation sensor and a measurement unit according to an embodiment of the present disclosure;
FIG. 20 is a sectional view showing a state of a needle of the treatment device according to the embodiment of the present disclosure before entering an inlet;
FIG. 21 is a sectional view showing a state of the needle of the treatment device according to the embodiment of the present disclosure before being withdrawn from a needle guide; and
FIG. 22 is a sectional view showing a state in which a needle according to an embodiment of the present disclosure is withdrawn from a needle guide.

### DETAILED DESCRIPTION

Reference will now be made in detail to embodiments of the present disclosure, examples of which are illustrated in the accompanying drawings, wherein like reference numerals refer to like elements throughout. For convenience of description, a medical treatment device will be described as an example. In the drawings, the thickness of each line or size of each component may be exaggerated for convenience of description and clarity. Also, terms, which will be described below, may be defined considering functions of the present disclosure, and may vary according to usual practice or intention of users or operators. Consequently, such terms may be defined based on the specification.

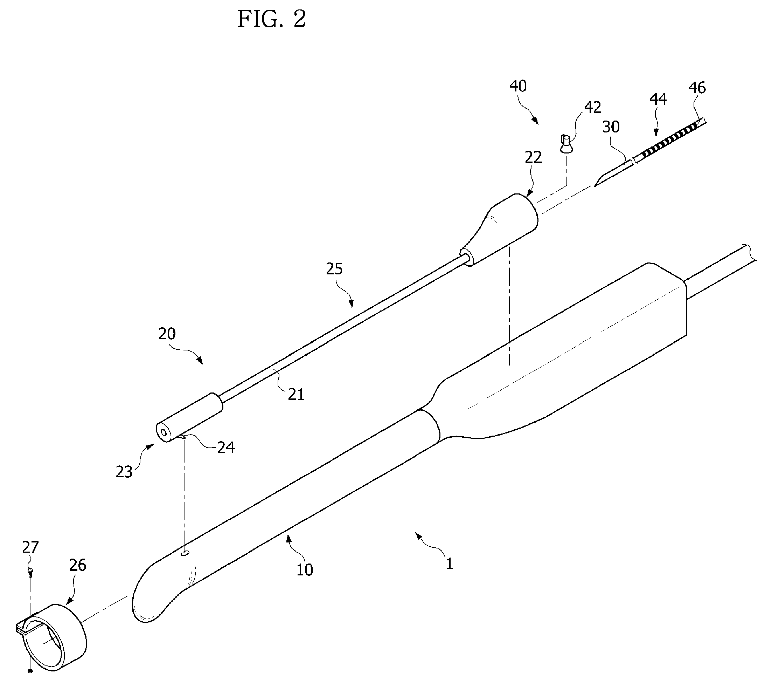

As shown in FIG. 2, one embodiment of the present disclosure includes a treatment device 1. The treatment device 1 includes a probe 10 for acquiring an image of a target (not shown) using ultrasonic waves. A needle guide 20 is provided in the probe 10. A needle 30 is movable along the needle guide 20. A measurement unit 40 senses a movement distance of the needle 30 in a noncontact fashion, and a controller 40 calculates the movement distance of the needle 30 based on a value measured by the measurement unit 40.

Various ultrasonic diagnostic devices may be used as the probe 10 as long as the ultrasonic diagnostic devices acquire an image of a target using ultrasonic waves.

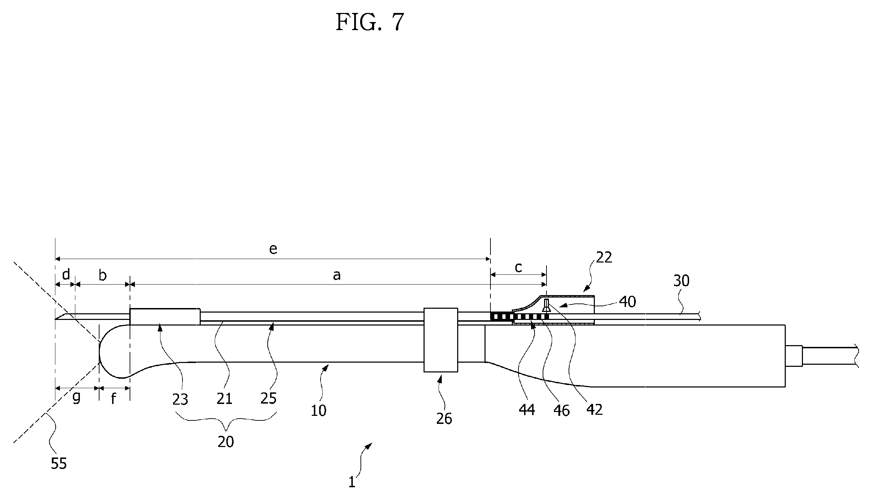

In certain embodiments, the probe 10 transmits an ultrasonic signal, such as the signal 55 as shown in FIG. 7, to a target (not shown) and receives an ultrasonic signal reflected from the target to acquire an ultrasonic image of the target.

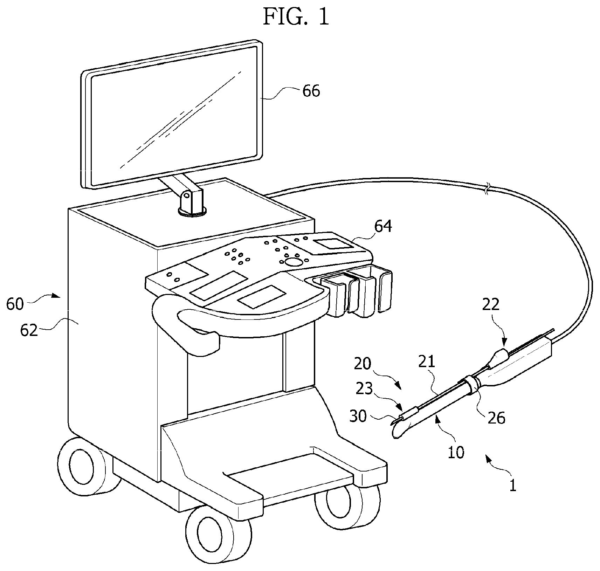

As illustrated in FIG. 1, the probe 10 can be connected to a main unit 60 so that the ultrasonic signal received by the probe 10 is transmitted to the main unit 60.

In this embodiment, the main unit 60 includes a main body 62, in which various kinds of equipment are installed. A manipulation panel 64 is connected to the main body 62 to allow a user to input a manipulation signal. A display unit 66 displays the signal received by the probe 10.

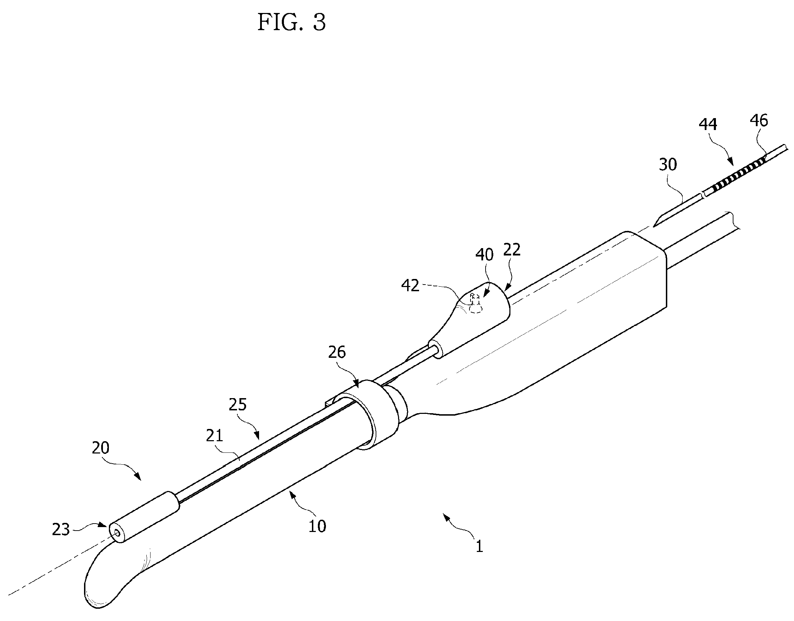

The needle guide 20 is provided along the probe 10 in the embodiment shown in FIG. 3. The needle guide 20 may be formed in any shape suitable to allow for the needle guide 20 to guide movement of the needle 30.

In one embodiment, the needle guide 20 includes a guide pipe 21 provided along the probe 10 to define a passage therein, an inlet 22 provided at one side (right side in FIG. 2) of the guide pipe 21, and an outlet 23 provided at the other side (left side in FIG. 2) of the guide pipe 21.

The inlet 22 has a diameter greater than that of the guide pipe 21 so that the needle 30 easily enters the guide pipe 21. A portion of the inlet 22 connected to the guide pipe 21 can be inclined to guide movement of the needle 30.

As shown in the embodiment of FIG. 2, a hook 24 protruding from the outlet 23 catches the probe 10 to fix the outlet 23 to the probe 10.

A connection member 26 connected to the inlet 22 or the guide pipe 21 is formed in the shape of a band. In this embodiment, the connection member 26 is provided to wrap the probe 10 and the guide pipe 21 of the needle guide 20. Opposite ends of the connection member 26 are fixed by a fastening bolt 27.

To operate the device according to one embodiment, the needle 30 moves along the guide pipe 21 through the inlet 22 and is withdrawn from the needle guide 20 through the outlet 23 to insert the needle 30 into a target.

The needle 30 may be formed in any shape suitable to allow for the needle 30 to move along the needle guide 20 to sample or treat a target.

The needle 30 may include an insertion part, which is inserted into a target, e.g. a human body, to be measured, and an installation part, at which the measurement unit 40 is installed, to avoid infection during measurement.

That is, the insertion part and the installation part of the needle 30 are separated from each other so that the installation part is disinfected or replaced, thereby preventing infection during measurement.

Any noncontact sensor may be used as the measurement unit 40 as long as the sensor is suitable for measuring a movement distance of the needle in a noncontact fashion.

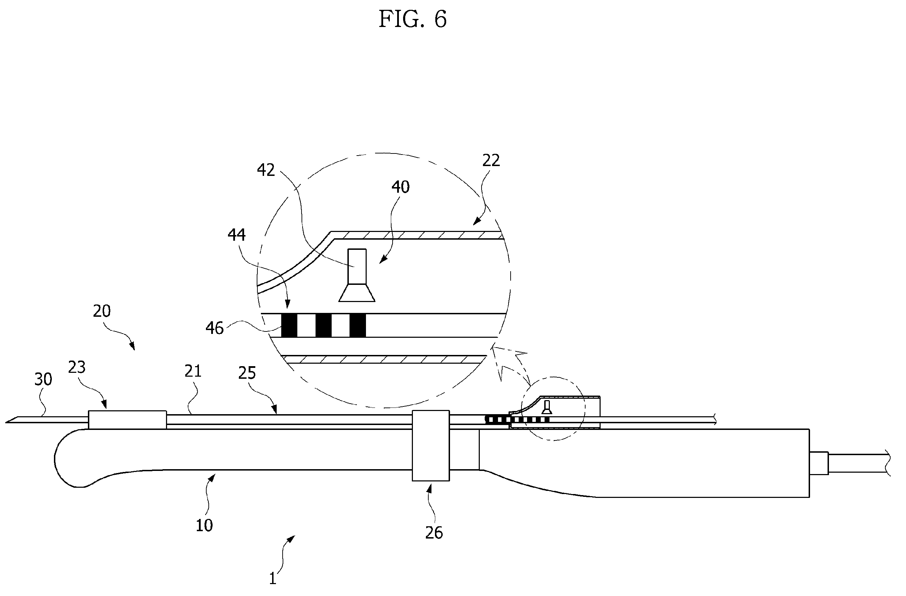

In an embodiment in which the measurement unit 40 is provided in the inlet 22, germs are prevented from moving along the needle 30 to provide more sanitary test environments since external contamination sources and the front end (left side in FIG. 6) of the needle 30 are relatively distant from the inlet 22.

In an embodiment in which the inlet 22 has too small a space to receive the measurement unit 40, the measurement unit 40 may be provided in the middle 25 of the needle guide 20 between the inlet 22 and the outlet 23 to improve space utilization.

In certain embodiments, the measurement unit 40 may weigh 1 kg or less. Since the measurement unit 40 is relatively light, a user may easily manipulate the treatment device 1 including the measurement unit 40.

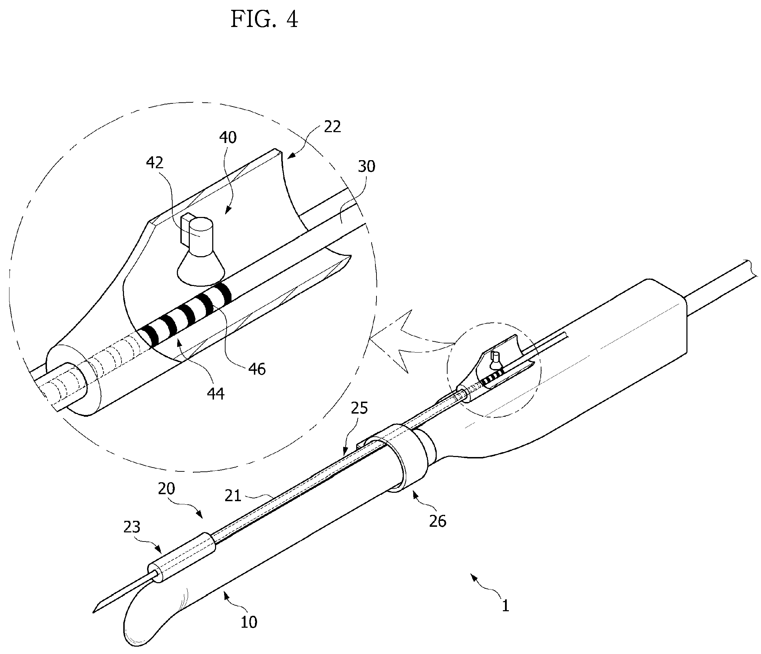

In embodiments shown in FIGS. 4-7, the measurement unit 40 includes a first indicator 44 formed at the outside of the needle 30 for indicating length information and a camera 42 for capturing and transmitting the first indicator 44 to the controller 50 as an image signal.

The first indicator 44 may have a color or pattern varying according to the length information of the needle 30 so that a user recognizes an outward movement distance of the needle 30 through the outlet 23 of the needle guide 20.

The first indicator 44 may indicate a measurement start part and a measurement end part. Also, the first indicator 44 may include distance information of a region at which the needle 30 is located.

For example, when the front end of the needle 30 moves 3cm outward from the outlet 23, distance information showing 3cm of movement is indicated at the first indicator 44 captured by the camera 42, thereby confirming the movement distance of the needle 30 through the measurement of the first indicator 44.

In some embodiments, patterns showing front and rear ends of the first indicator 44 are different from each other. Consequently, the measurement start part and the measurement end part are easily confirmed based on such patterns.

In another embodiment, the first indicator 44 includes various colors, which change based on a movement distance of the needle 30. The colors are arranged at predetermined intervals, and the camera 42 captures and transmits the first indicator 44 provided at the outside of the needle 30 to the controller 50, thereby calculating the movement distance of the needle 30.

In one embodiment, the first indicator 44 may be variously modified as long as the first indicator 44 indicates information to calculate the movement distance of the needle 30 based on changed colors or patterns.

The first indicator 44 may be attached to the outside of the needle 30 as a sticker. Alternatively, the first indicator 44 may be attached to the outside of the needle 30 using various methods, such as painting or processing.

In another embodiment, the first indicator 44 includes indicator bands 46, which are provided at the outside of the needle 30 at a predetermined interval.

For example, in one embodiment, a start part of the first indicator 44 is a position measured by the camera 42 immediately before the needle 30 is withdrawn from the outlet 23, and an end part of the first indicator 44 is a position measured by the camera 42 after the needle 30 is inserted into a target. Such positions of the first indicator 44 correspond to a movement distance of the needle withdrawn from the outlet 23.

Any image capturing device may be used as the camera 42 as long as the capturing devices are suitable for capturing an image of the first indicator 44 and transmit an image signal to the controller 50.

The camera 42 may be provided at various positions. For example, the camera 42 may be provided inside the inlet 22 or in the middle 25 located between the inlet 22 and the outlet 23.

An image captured by the camera 42 may be transmitted to the controller 50 in a wired or wireless fashion. Also, power may be supplied to the camera 42 in a wired or wireless fashion.

The camera 42 may capture a set number of frames per second and transmit the captured frames to the controller 50. Alternatively, the camera 42 may analyze a movement distance of the needle 30 based on the number of frames and transmit only a measured value to the controller 50.

In other embodiments, a transparent protective film (not shown) may be provided between the camera 42 and the needle 30 to protect the camera 42.

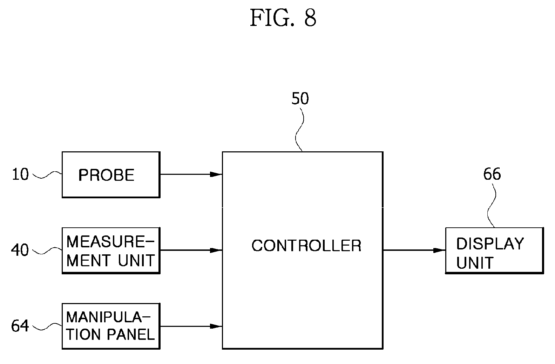

As shown in FIGS. 1 and 8, the controller 50 according to this embodiment calculates a movement distance of the needle 30 based on a value measured by the measurement unit 40, and an ultrasonic image signal measured by the probe 10 is also transmitted to the controller 50.

As shown in the flow chart in FIG. 8, the manipulation panel 64, which allows a user to input a manipulation signal, is connected to the controller 50 to transmit a signal to the controller 50. The controller 50 synthesizes and outputs signals from the probe 10, the measurement unit 40 and the manipulation panel 64 to the display unit 66.

The controller 50 may be disposed at one or more selected from among the probe 10, the needle guide 20 and the main unit 60.

Hereinafter, the operation of the treatment device 1 according to certain embodiments will be described in detail with reference to the accompanying drawings.

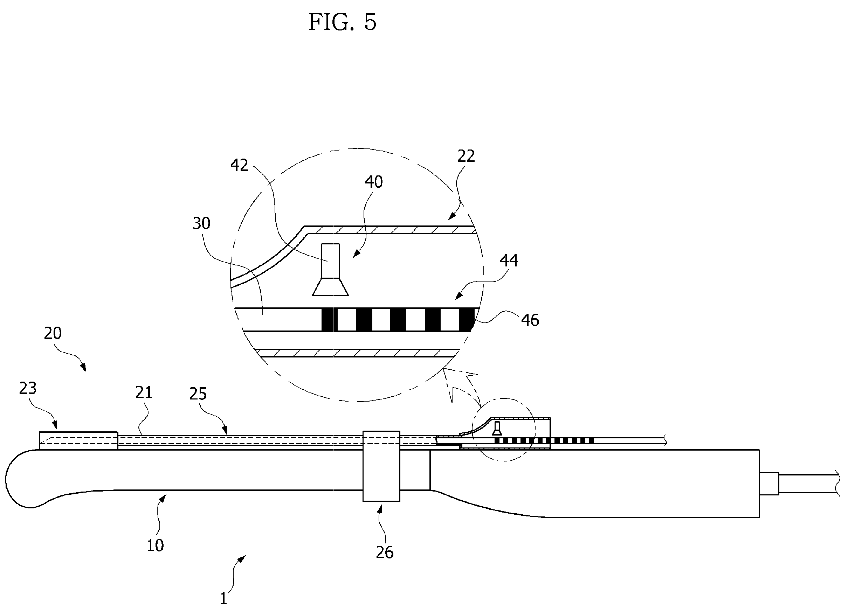

As shown in FIG. 3, the needle 30 is moved toward the inlet 22 of the needle guide 20. Subsequently, as shown in FIG. 5, the needle 30 is inserted through the inlet 22 and is moved along the guide pipe 21.

When the front end of the needle 30 is placed in the outlet 23 of the needle guide 20, the indicator bands 46 of the first indicator 44 are captured by the camera 42.

As the needle 30 is withdrawn through the outlet 23, as shown in FIGS. 4 and 6, the first indicator 44 moves with the needle 30.

The camera 42 captures the first indicator 44 and transmits images on a per frame basis to the controller 50. The controller 50 compares an image pattern signal transmitted from the camera 42 with a stored image pattern signal to measure a movement distance of the needle 30.

In other embodiments, the number of the indicator bands 46 captured by the camera 42 may be multiplied by an interval between the indicator bands 46 to measure the movement distance of the needle 30.

The controller 50 calculates the movement distance of the needle 30 based on a value measured by the camera 42 and provides the result to a user. The controller 50 may provide the movement distance of the needle 30 to the user through the display unit 66. Alternatively, a speaker may be used to provide the movement distance of the needle 30 to the user via audio signals.

The user confirms the movement distance of the needle 30 through the display unit 66, thereby more safely sampling or treating a target.

Various calculation expressions and methods may be used to calculate the length of the needle 30 inserted into a human body using the treatment device 1 according to certain embodiments.

In the embodiment as shown in FIG. 7, the distance from the front end (left side in FIG. 7) of the outlet 23 to the camera 42 is expressed by a symbol 'a'. A beam irradiation surface 55, to which ultrasonic waves are irradiated to acquire an image, is provided at the front (left side in FIG. 7) of the probe 10. The distance from the interface between the beam irradiation surface 55 and the needle 30 to the front end of the outlet 23 is expressed by a symbol 'b'.

The distance from a zero position (left side in FIG. 7), which is a reference position of the first indicator 44, to the camera 42 of the measurement unit 40 to sense a position is expressed by a symbol 'c'. A value of 'c' may be acquired through the measurement unit 40. The distance from the zero position, which is the reference position of the first indicator 44, to the front end (left side in FIG. 7) of the needle 30 is expressed by a symbol 'e'.

Values of 'a', 'b' and 'e' may be kinematically calculated or acquired through real measurement. These values are stored in the controller 50. The value of 'c' may be acquired through the operation of the measurement unit 40 according to movement of the needle 30. This value is transmitted to the controller 50.

The distance 'd' from the interface between the beam irradiation surface 55 and the needle 30 to the front end (left side in FIG. 7) of the needle 30 may be acquired based on these values. That is, a symbol 'd' is a length of the needle 30 displayed only on the beam irradiation surface 55. A value of 'd' may be acquired using the following expression: 'd=(c+e)-(a+b)'.

The front end (left side in FIG. 7) of the probe 10 is a part contacting a target to be tested, i.e. a human body. The distance from the front end of the probe 10 to the front end of the needle 30 is a length of the needle 30 inserted into the human body, which is expressed by a symbol 'g'.

The length from the front end of the probe 10 to the front end of the outlet 23 may be expressed by a symbol 'f'. A value of 'f' may be kinematically calculated or acquired through real measurement. Consequently, the length g of the needle inserted into the human body is calculated using the following expression: 'g=(b+d)-f'.

Values of 'b' and 'f' are stored in the controller. The expression to calculate the value of 'd' is substituted into the expression to calculate the value of 'g' to derive the following expression: 'g=(c+e)-(a+f)'.

These calculation expressions and methods may be used in a mechanical measurement unit operating in a contact fashion as well as optical measurement units 40, 70, 80, 90 and 100 (as shown in FIGS. 8-16) to measure the movement distance of the needle 30.

Hereinafter, a treatment device 2 according to another embodiment of the present disclosure will be described with reference to the accompanying drawings. For convenience of description, components of this embodiment identical in construction and operation to those of the previous embodiment shown in FIGS. 1 to 8 are denoted by the same reference numerals, and a detailed description thereof will be omitted.

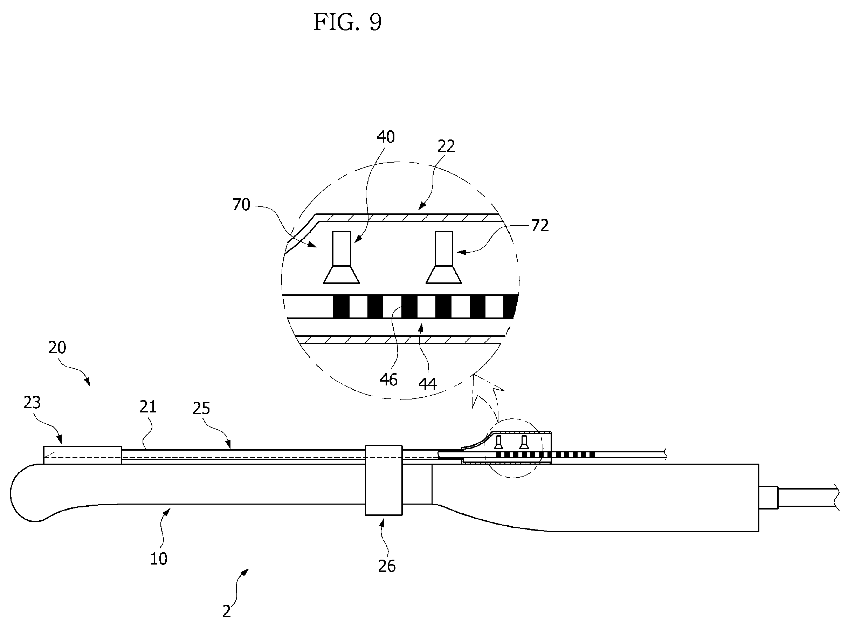

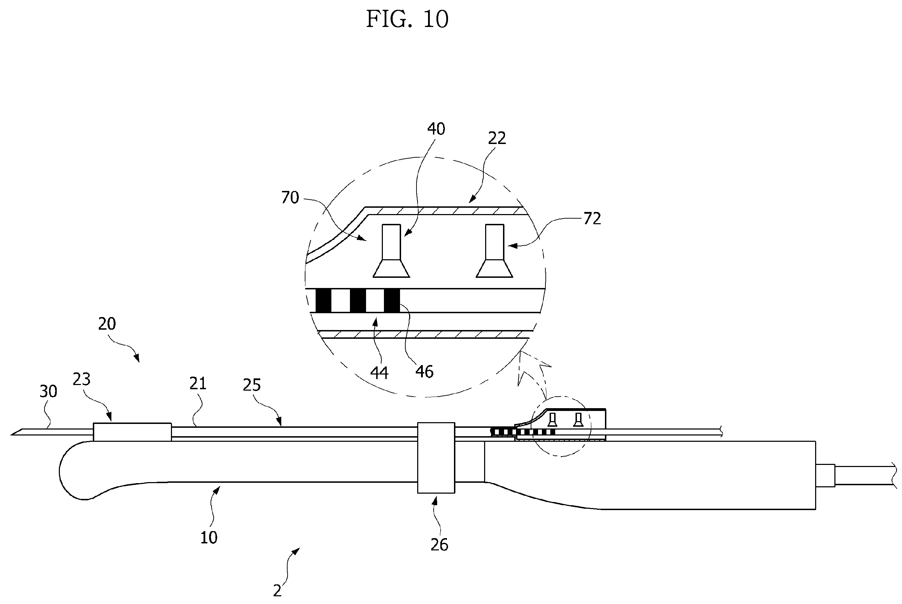

According to the embodiments shown in FIGS. 9 and 10, a measurement unit 70 of the treatment device 2 includes a first indicator 44 for indicating length information at the outside of a needle 30, a camera 42 for capturing and transmitting the first indicator 44 to a controller 50 as an image signal, and a first optical sensor 72 for measuring movement of the first indicator 44 by transmitting and receiving light.

The first optical sensor 72 includes a light emitting part for irradiating light to the needle 30 and a light receiving part for sensing light reflected from the needle 30.

Any suitable light emitting devices, including a light emitting diode (LED), may be used as the light emitting part of the first optical sensor 72. An electric device to receive and convert light reflected from the needle 30 or the first indicator 44 into an electric signal may be used as the light receiving part of the first optical sensor 72.

An optical sensor used in an optical mouse may be used as the first optical sensor 72. The optical sensor may cooperate with the camera 42 to measure a movement distance of the needle 30.

A value measured by the first optical sensor 72 may be transmitted to the controller 50. The value measured by the first optical sensor 72 may be analyzed by a digital signal processing chip (DSPC) to calculate a movement distance of the needle 30. The value themn may be transmitted to the controller 50.

In this embodiment, the treatment device 2 measures a movement distance of the needle 30 using the camera 42 and the first optical sensor 72, thereby more accurately measuring the movement distance of the needle 30.

In a state in which the front end of the needle 30 is placed in the outlet 23 of the needle guide 20, as shown in FIG. 9, the first indicator 44 is captured by the camera 42.

As the needle 30 is withdrawn through the outlet 23, as shown in FIG. 10, the first indicator 44 moves with the needle 30.

The camera 42 captures the first indicator 44 and transmits images on a per frame basis to the controller 50. The first optical sensor 72 measures a movement distance of the needle 30 and transmits a measured value to the controller 50.

The controller 50 calculates the movement distance of the needle 30 using the number of indicator bands 46 captured by the camera 42 and an interval between the indicator bands 46.

In some embodiments, the movement distance of the needle 30 is calculated based on a value measured by the first optical sensor 72 and is set to an average of the measured values acquired by the camera 42 and the first optical sensor 72.

The controller 50 may provide the movement distance of the needle 30 to a user through a display unit 66. Alternatively, an additional speaker may be used to provide the movement distance of the needle 30 to the user.

The user easily confirms the movement distance of the needle 30 through the display unit 66, thereby safely sampling or treating a target.

Hereinafter, a treatment device 3 according to another embodiment of the present disclosure will be described with reference to the accompanying drawings. For convenience of description, components of this embodiment identical in construction and operation to those of the previous embodiment shown in FIGS. 1 to 8 are denoted by the same reference numerals, and a detailed description thereof will be omitted.

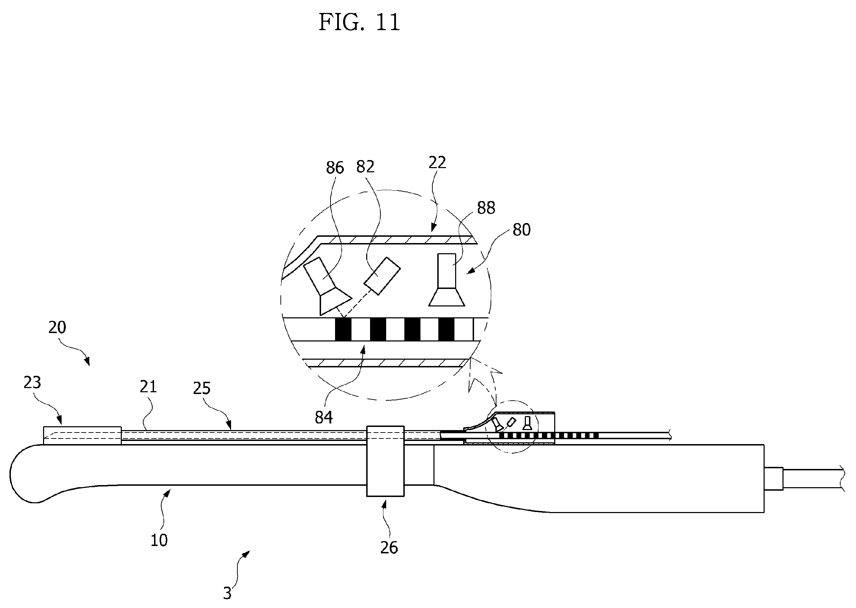

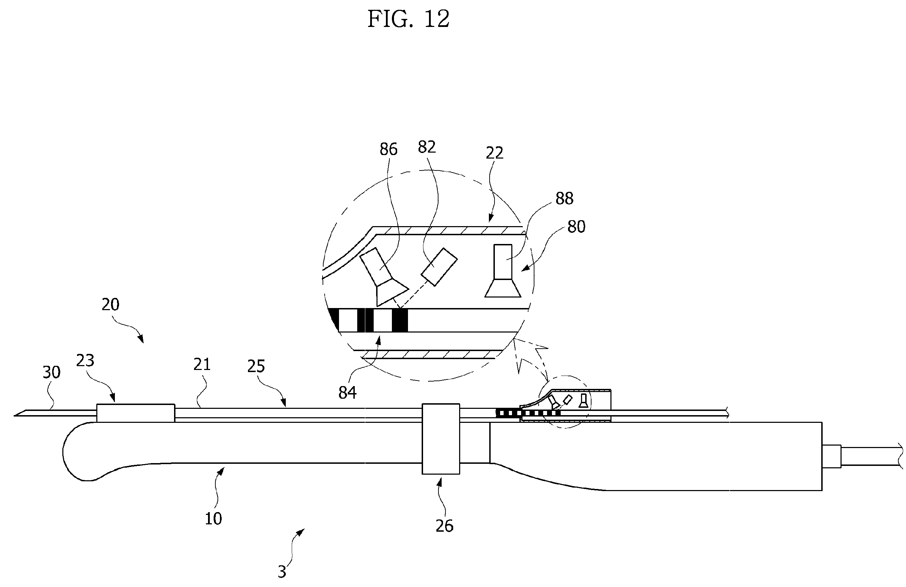

In the embodiment shown in FIGS. 11 and 12, a measurement unit 80 of the treatment device 3 includes an irradiation unit 82 for irradiating light of a predetermined wavelength at a predetermined time interval, a second indicator 84 having a pattern formed of a paint, and reflecting the light of the predetermined wavelength among the light irradiated from the irradiation unit 82, an optical sensor 86 for sensing the light of the wavelength reflected from the second indicator, and a second optical sensor 88 for measuring movement of the second indicator 84.

The measurement unit 80 may be provided in an inlet 22, through which a needle 30 is inserted into a needle guide 20, in a probe 10 adjacent to the inlet 22, or the middle 25 of the needle guide 20.

Any irradiation device may be used as the irradiation unit 82 as long as the irradiation device is suitable for irradiating light of a specific wavelength to the needle 30.

In one embodiment, the irradiation unit 82 may continuously irradiate light of a predetermined wavelength or irradiate light of a predetermined wavelength at a predetermined time interval. The second indicator 84 includes a paint reflecting light of a specific wavelength. The second indicator 84 is provided at the rear side (right side in FIG. 10) of the needle 30 to detour around an insertion part of the needle 30, which is inserted into a human body.

The second indicator 84 may be provided at the outside of the needle 30 using various methods, such as printing, application and attachment. The paint is provided at the second indicator 84 at a predetermined interval to repeatedly reflect and block light irradiated from the irradiation unit 82 toward the optical sensor 86.

The light reflected from the second indicator 84 is received by the optical sensor 86 and converted into an electric signal, which is transmitted to the controller 50. The movement distance of the needle 30 may be measured using the irradiation unit 82, the second indicator 84 and the optical sensor 86. In addition, the second optical sensor 88 is provided to measure the movement distance of the needle 30.

In the embodiment shown in FIG. 11, the second optical sensor 88 according to one embodiment has the same construction and operation as the first optical sensor 72 according to the previous embodiment shown in FIGS. 9 and 10, and therefore, a detailed description thereof will be omitted.

In a state in which the front end of the needle 30 is placed in the outlet 23 of the needle guide 20, as shown in FIG. 11, the irradiation unit 82 irradiates light of a specific wavelength to the second indicator 84. The light of the specific wavelength reflected from the second indicator 84 is received by the optical sensor 86 and converted into an electric signal, which is transmitted to the controller 50.

As the needle 30 is withdrawn through the outlet 23, as shown in FIG. 12, the second indicator 84 moves with the needle 30. According to the movement of the second indicator 84, the light from the irradiation unit 82 is repeatedly reflected and non-reflected to the optical sensor 86. The optical sensor 86 generates an electric signal based on when the light is received and when the light is not received.

The controller 50 analyzes the signal of the second indicator 84 measured by the optical sensor 86 to calculate the movement distance of the needle 30.

In one embodiment, the movement distance of the needle 30 is calculated based on a value measured by the second optical sensor 88 and is set to an average of the measured values acquired by the optical sensor 86 and the second optical sensor 88. Hereinafter, a treatment device 4 according to another embodiment of the present disclosure, however, not forming part of the present invention, will be described with reference to the accompanying drawings. For convenience of description, components of this embodiment identical in construction and operation to those of the previous embodiment shown in FIGS. 1 to 8 are denoted by the same reference numerals, and a detailed description thereof will be omitted.

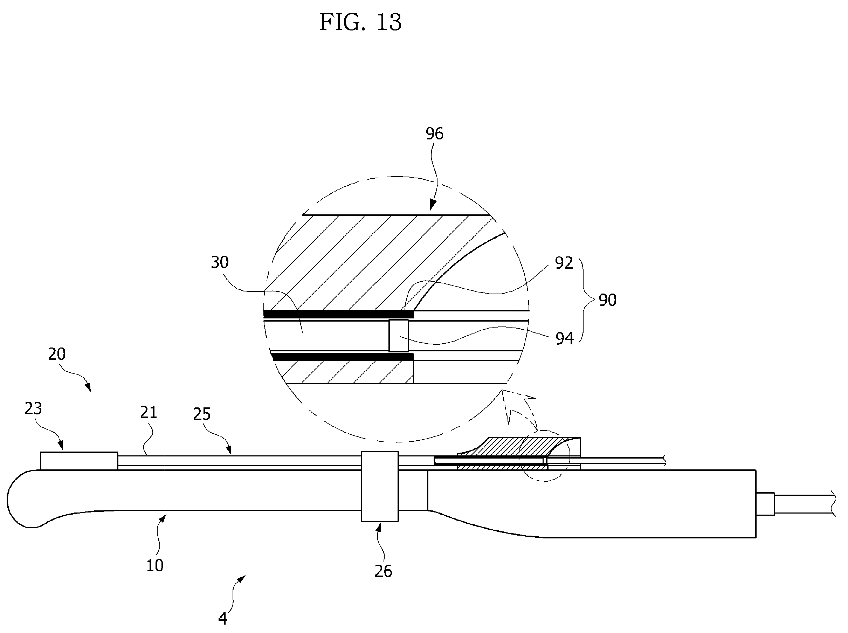

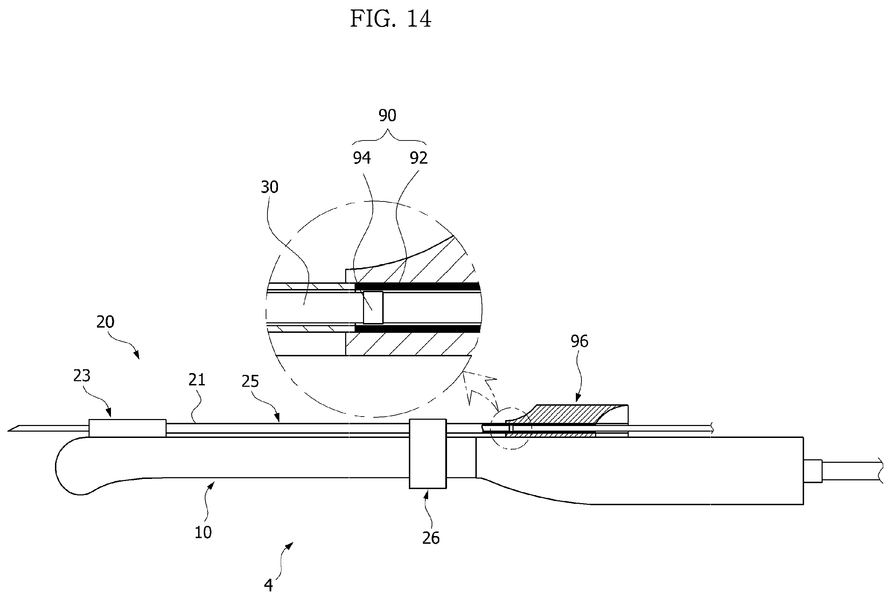

In the embodiment shown in FIGS. 13 and 14, a measurement unit 90 of the treatment device 4 includes a magnetic field generation unit 92 for generating a magnetic field at a region through which a needle 30 extends and a coil sensor 94 interlocked with the needle 30 for sensing change of the magnetic field caused while passing through the magnetic field generation unit 92.

The magnetic field generation unit 92 is provided in a needle guide 20 or a probe 10 for generating a magnetic field in an inlet 96 of the needle guide 20.

The magnetic field generation unit 92 may be formed in various shapes, such as a cylindrical shape. The magnetic field generation unit 92 generates different magnetic fields depending upon positions at which the coil sensor 94, which moves with the needle 30, is located.

The coil sensor 94, which moves with the needle 30, is connected to the controller 50 to measure change of the magnetic field caused while passing through the magnetic field generated by the magnetic field generation unit 92 and to transmit the measured change of the magnetic field to the controller 50.

Any suitable electromagnetic device may be used as the magnetic field generation unit 92 and the coil sensor 94 as long as power is supplied to the electromagnetic device in a wired or wireless fashion so that the electromagnetic device generates a magnetic field.

When the front end of the needle 30 is placed in the outlet 23 of the needle guide 20, as shown in FIG. 13, the coil sensor 94 is introduced into the magnetic field generation unit 92 to sense change of a magnetic field.

The coil sensor 94 senses the change of the magnetic field and transmits a measured value to the controller 50. Consequently, the controller 50 recognizes the positions of the coil sensor 94 and the needle 30 at which the coil sensor 94 is provided.

As the needle 30 is withdrawn through the outlet 23, as shown in FIG. 14, the coil sensor 94 moves with the needle 30. The coil sensor 94 measures magnetic fields changed depending upon the position thereof while moving along the inside of the magnetic field generation unit 92. The measured value is converted into an electric signal, which is transmitted to the controller 50.

The controller 50 analyzes the magnetic field change signal measured by the coil sensor 94 to calculate the movement distance of the needle 30 and to provide the result to a user through a display unit 66.

Hereinafter, a treatment device 5 according to another embodiment of the present disclosure, however, not forming part of the present invention, will be described with reference to the accompanying drawings. For convenience of description, components of this embodiment identical in construction and operation to those of the previous embodiment shown in FIGS. 1 to 8 are denoted by the same reference numerals, and a detailed description thereof will be omitted.

A measurement unit 100 of the treatment device 5 measures a movement distance of a needle 30 using electromagnetic resonance (EMR).

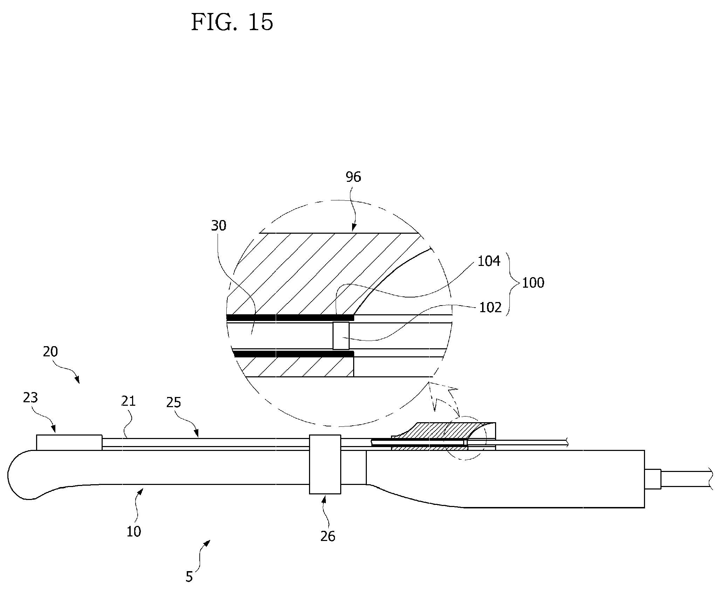

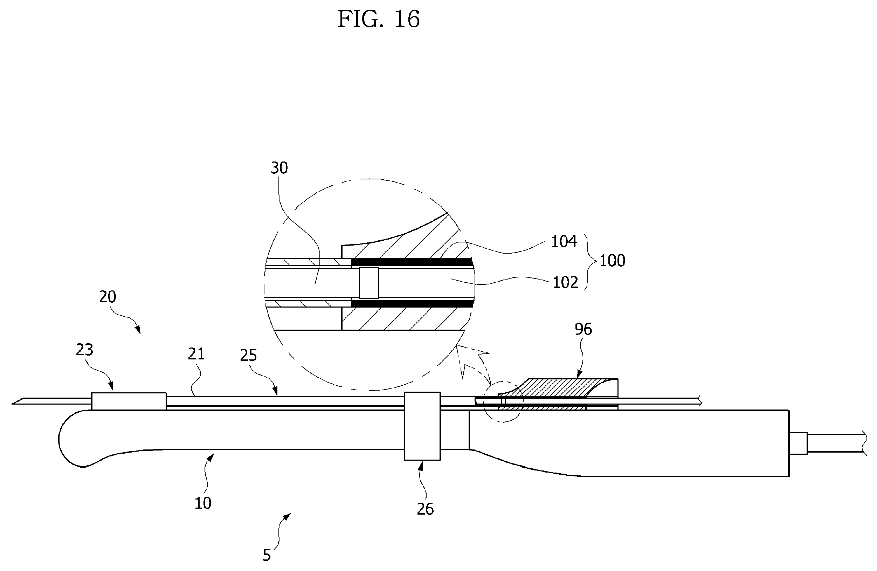

In the embodiment shown in FIGS. 15 and 16, the measurement unit 100 includes a resonance circuit 102 interlocked with the needle 30 and a resonance sensor 104, provided at a region through which the needle 30 extends, to transmit and receive electric waves to and from the resonance circuit 102 to measure the movement distance of the needle 30.

The resonance sensor 104 is provided in a needle guide 20 or a probe 10. Any suitable electronic device may be used as the resonance sensor 104 as long as the electronic device transmits and receive an electromagnetic signal to and from the resonance circuit 102.

The resonance sensor 104 may be formed in any suitable shapes. One example of a suitable shape is a cylindrical shape.

Power is supplied to the resonance sensor 104, which is fixedly provided. Power is not supplied to the resonance circuit 102, which moves with the needle 30.

The resonance sensor 104 includes a loop coil and a transmitting and receiving swap circuit. The resonance circuit 102 includes a coil and a condenser.

The resonance sensor 104 receives electric waves generated from the resonance circuit 102, which moves with the needle 30, to measure the movement distance of the needle 30. A value measured by the resonance sensor 104 is converted into an electric signal, which is transmitted to the controller 50.

When the front end of the needle 30 is placed in the outlet 23 of the needle guide 20, the resonance circuit 102 is introduced into the resonance sensor 104.

In other embodiments, the resonance sensor 104 is operated in a transmitting mode and in a receiving mode. In the transmitting mode, electric waves generated by the loop coil of the resonance sensor 104 are received by the coil of the resonance circuit 102, and the condenser is charged with induced voltage generated by the electric waves. In the receiving mode, no electric waves are transmitted from the loop coil, and the resonance circuit 102 performs free oscillation.

As current flows in the coil of the resonance circuit 102, predetermined electric waves are generated by the coil. Induced voltage is generated by the loop coil of the resonance sensor 104, which receives the electric waves. A value measured by the resonance sensor 104 is converted into an electric signal, which is transmitted to the controller 50.

The resonance sensor 104 receives different electric waves caused by the resonance circuit 102, which moves in the resonance sensor 104 along with the needle 30. Such a signal is transmitted to the controller 50, which calculates the movement distance of the resonance circuit 102 and the needle 30, in which the resonance circuit 102 is provided. The result is provided to a user through a display unit 66.

As described above, the treatment device 1, 2, 3, 4 or 5 senses the movement distance of the needle 30 in a noncontact fashion. Consequently, contamination of the needle 30 is prevented, and the movement distance of the needle 30 is accurately measured, thereby improving stability of biopsy.

Also, the measurement unit 40, 70, 80, 90 or 100 is selectively provided in the needle 30, the needle guide 20 and the probe 10. Consequently, no additional space is needed, thereby improving space utilization.

A treatment device according to an embodiment of the present disclosure may further include a needle sensing unit 120 to determine whether the needle 30 has reached a reference position 110. The measurement unit 40, 70, 80, 90 or 100 is driven based on the determination of the needle sensing unit 120 to measure the movement distance of the needle 30.

The reference position means a position at which the measurement unit begins measurement when the needle reaches the position.

Hereinafter, the treatment further including the needle sensing unit 120 will be described in detail. For convenience of description, this embodiment includes the measurement unit 40 of the previous embodiment shown in FIGS. 1 to 8.

In this embodiment, the reference position 110 is provided below the camera 42. When the first indicator 44, which moves with the needle 30, reaches the reference position 110, the camera 42 is driven.

A contact type sensor or a noncontact type sensor may be used as the needle sensing unit 120, which drives the camera 42. In one embodiment, the needle sensing unit 120 includes a recognition member 122 provided at the outside of the needle 30 and a recognition sensor 124 to recognize the recognition member 122 to drive the needle sensing unit 120.

The recognition member 122 may be formed in any suitable shape as long as the recognition member 122 is recognized by the needle sensing unit 120.

In this embodiment, the recognition member 122 is disposed at the outside of the needle 30, and is band-shaped. Consequently, the recognition member 122 is easily recognized by the needle sensing unit 120 irrespective of directivity of the needle 30.

The recognition sensor 124 is operated in a contact or noncontact fashion to recognize the recognition member 122. In this embodiment, the recognition sensor 124 recognizes the recognition member 122 through transmission and reception of light. Consequently, operational reliability of the recognition sensor 124 is secured although the recognition sensor 124 is repeatedly used.

The distance between the recognition sensor 124 and the camera 42 is equal to that between the recognition member 122 and the first indicator 44. When the recognition member 122 is recognized by the recognition sensor 124, therefore, the camera 42 measures the first indicator 44 having reached the reference position 110 in an initialized state.

As shown in FIG. 8, the controller 50 calculates a movement distance of the needle 30 based on a value measured by the measurement unit 40, and an ultrasonic image signal measured by the probe 10 is also transmitted to the controller 50.

Also, the manipulation panel 64, which allows a user to input a manipulation signal, is connected to the controller 50 to transmit a signal to the controller 50. The controller 50 synthesizes and outputs signals from the probe 10, the measurement unit 40 and the manipulation panel 64 to the display unit 66.

The controller 50 may be disposed at one or more selected from among the probe 10, the needle guide 20 and the main unit 60.

Hereinafter, the operation of the treatment device according to another embodiment of the present disclosure will be described in detail with reference to the accompanying drawings.

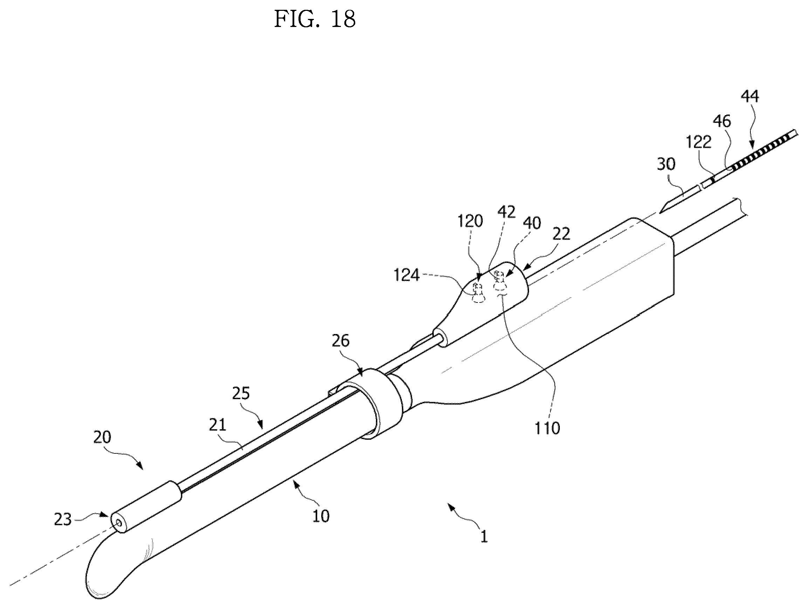

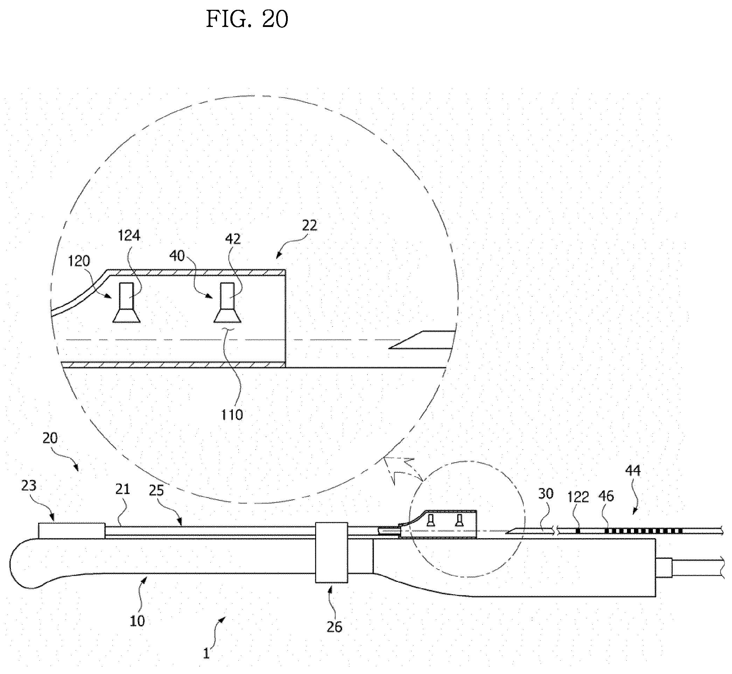

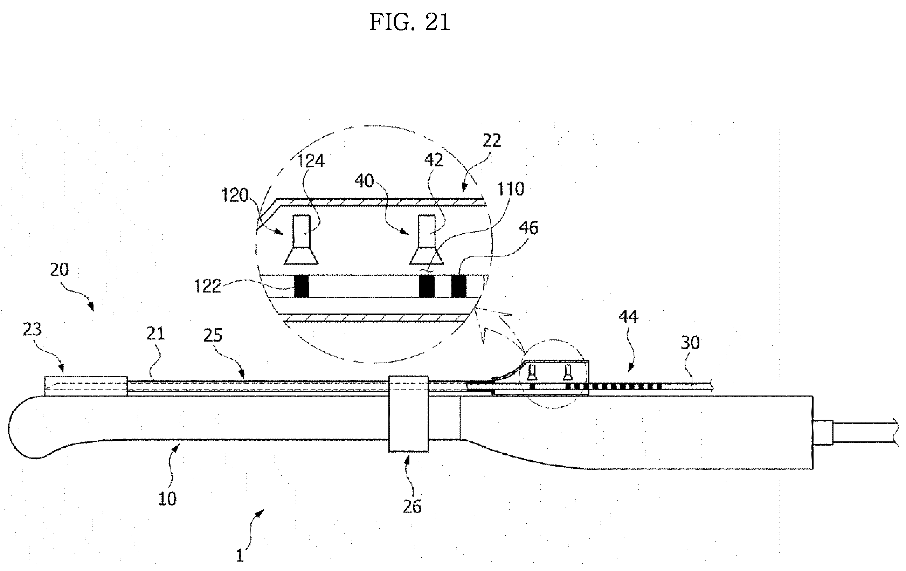

As shown in FIG. 18 and 20, the needle 30 is moved toward the inlet 22 of the needle guide 20. Subsequently, as shown in FIG. 21, the needle 30 is inserted through the inlet 22 and is moved along the guide pipe 21.

When the recognition member 122 is recognized by the recognition sensor 124 in a state in which the front end of the needle 30 is placed in the outlet 23 of the needle guide 20, the needle sensing unit 120 transmits a measured signal to the controller 50, which transmits a drive signal to the camera 42 of the measurement unit 40.

Alternatively, the recognition sensor 124 may directly transmit a drive signal to the camera 42 to drive the camera, and the indicator bands 46 of the first indicator 44 are captured by the camera 42.

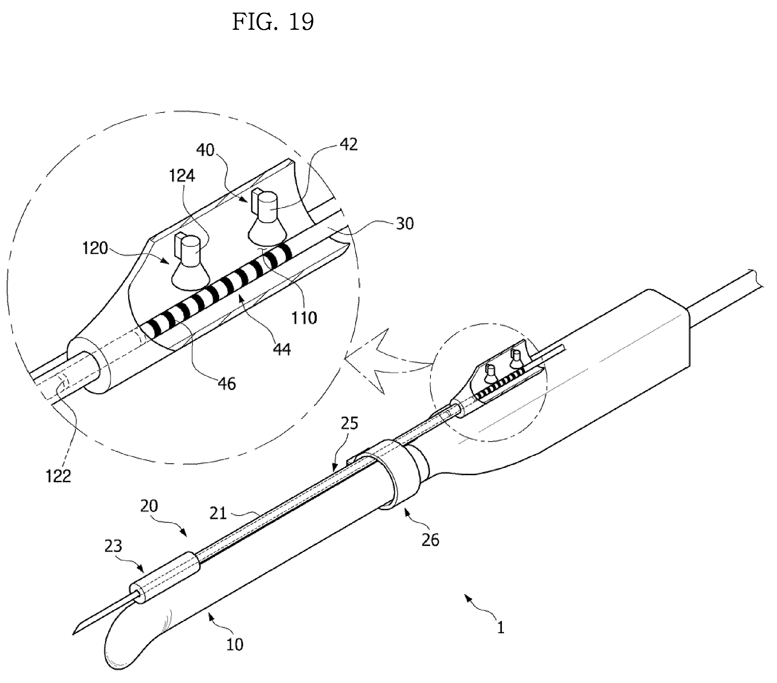

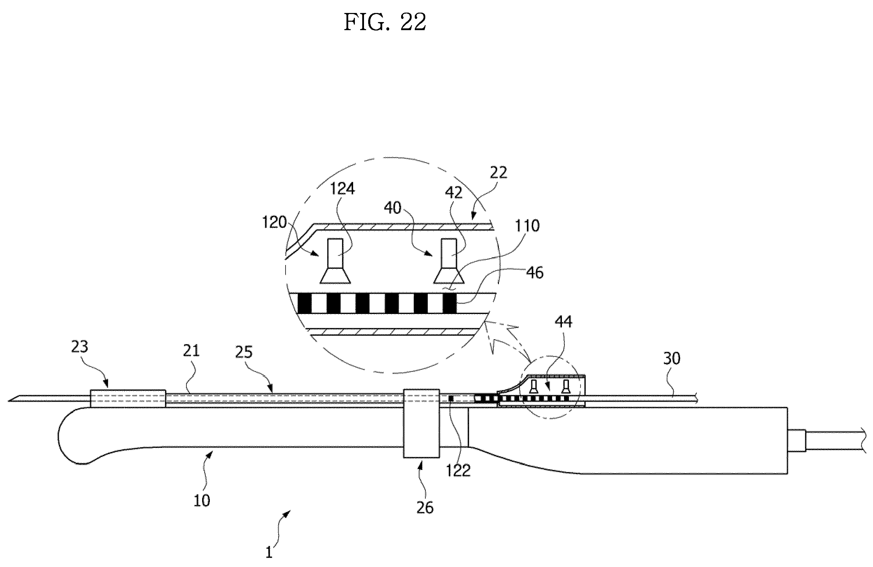

As the needle 30 is withdrawn through the outlet 23, as shown in FIGS. 19 and 22, the first indicator 44 moves with the needle 30.

The camera 42 captures the first indicator 44 and transmits images on a per frame basis to the controller 50. The controller 50 compares an image pattern signal transmitted from the camera 42 with a stored image pattern signal to measure a movement distance of the needle 30.

Alternatively, the number of the indicator bands 46 captured by the camera 42 may be multiplied by an interval between the indicator bands 46 for measuring the movement distance of the needle 30. The controller 50 calculates the movement distance of the needle 30 based on a value measured by the camera 42 and provides the result to a user. The controller 50 may provide the movement distance of the needle 30 to the user through the display unit 66. Alternatively, an additional speaker may be used to provide the movement distance of the needle 30 to the user via audio signals.

The user easily confirms the movement distance of the needle 30 through the display unit 66, thereby more safely sampling or treating a target.

Any suitable calculation expressions and methods may be used to calculate the length of the needle 30 inserted into a human body using the treatment device according to this embodiment. The method previously explained with reference to FIG. 7 may be used.

In the treatment device with the above-stated construction, the measurement unit 40 measures length information of the needle 30 based on the operation of the needle sensing unit 120 to determine whether the needle 30 has reached the reference position 110. Consequently, contamination of the needle 30 is prevented, and the movement distance of the needle 30 is accurately measured, thereby improving stability of biopsy.

Although this embodiment adopts the measurement unit 40 of the previous embodiment shown in FIGS. 1 to 8, the operation of the measurement units 40, 70, 80, 90 and 100 may be controlled by the needle sensing unit.

In the above, medical treatment devices were described; however, embodiments of the present disclosure may be applied to biopsy in other fields.

As is apparent from the above description, in the treatment device according to the embodiments of the present disclosure, the movement distance of the needle is sensed in a noncontact fashion, and the measurement unit measures length information of the needle based on the operation of the needle sensing unit to determine whether the needle has reached the reference position. Consequently, contamination of the needle is prevented, and the movement distance of the needle is accurately measured, thereby improving stability of biopsy.

Also, the measurement unit is selectively provided in the needle, the needle guide and the probe. Consequently, no additional space is needed, thereby improving space utilization.

## Claims

1. A treatment device comprising:
a probe (10) for acquiring an image of a target using ultrasonic waves;
a needle guide (20) provided in the probe (10); and
a needle (30) movable along the needle guide (20);
**characterized in that** the treatment device further comprises:
a measurement unit (40, 70, 80, 90, 100) for sensing a movement distance of the needle (30) without having a mechanical contact with the needle (30); and
a controller (50) for calculating the movement distance of the needle (30) based on a value measured by the measurement unit (40, 70, 80, 90, 100),
wherein the measurement unit (40) comprises: a first indicator (44) formed at an outside of the needle (30) for indicating length information; and a camera (42) for capturing the first indicator (44) and for transmitting the first indicator (44) to the controller (50) as an image signal.

2. The treatment device according to claim 1, wherein the first indicator (44) has a color or pattern varying according to the length information of the needle (30).

3. The treatment device according to claim 1, wherein the controller (50) is disposed at any one selected from the probe (10), the needle guide (20) and a main unit (60).

4. The treatment device according to claim 1, further comprising:
a needle sensing unit (120) for determining whether the needle (30) has reached a reference position,
wherein the measurement unit (40, 70, 80, 90, 100) is driven based on a determination of the needle sensing unit (120).

5. The treatment device according to claim 4, wherein the needle sensing unit (120) comprises:
a recognition member (122) provided at an outside of the needle (30); and
a recognition sensor (124) for driving the needle sensing unit (40, 70, 80) by recognizing the recognition member (122).

6. The treatment device according to claim 5, wherein the recognition member (120) is band-shaped.

7. The treatment device according to claim 5, wherein a distance between the recognition sensor (124) and the camera (42) is equal to a distance between the recognition member (122) and the first indicator (44).

## Patentansprüche

1. Behandlungsvorrichtung, welche folgendes aufweist:
Eine Sonde (10) zum Erlangen eines Bilds eines Ziels unter Verwendung von Ultraschallwellen;
eine Nadelführung (20), die in der Sonde (10) vorgesehen ist; und
eine Nadel (30), die entlang der Nadelführung (20) beweglich ist;
**dadurch gekennzeichnet, dass** die Behandlungsvorrichtung des Weiteren folgendes aufweist:
eine Messeinheit (40, 70, 80, 90, 100) zum Erfassen einer Bewegungsstrecke der Nadel (30), ohne einen mechanischen Kontakt mit der Nadel (30) zu haben; und
eine Steuereinrichtung (50) zum Berechnen der Bewegungsstrecke der Nadel (30) basierend auf einem von der Messeinheit (40, 70, 80, 90, 100) gemessenen Wert,
wobei die Messeinheit (40) folgendes aufweist:
eine erste Anzeige (44), die an einer Außenseite der Nadel (30) gebildet ist, um eine Längeninformation anzuzeigen; und eine Kamera (42) zum Erfassen der ersten Anzeige (44) und zum Übertragen der ersten Anzeige (44) zu der Steuereinrichtung (50) als ein Bildsignal.

2. Behandlungsvorrichtung nach Anspruch 1, wobei die erste Anzeige (44) eine Farbe oder ein Muster aufweist, das sich entsprechend der Längeninformation der Nadel (30) verändert.

3. Behandlungsvorrichtung nach Anspruch 1, wobei die Steuereinrichtung (50) an einem angeordnet ist, das aus der Sonde (10), der Nadelführung (20) und einer Haupteinheit (60) ausgewählt ist.

4. Behandlungsvorrichtung nach Anspruch 1, welche des Weiteren folgendes aufweist:
eine Nadelerfassungseinheit (120) zum Ermitteln, ob die Nadel (30) eine Referenzposition erreicht hat,
wobei die Messeinheit (40, 70, 80, 90, 100) basierend auf einer Ermittlung der Nadelerfassungseinheit (120) angetrieben wird.

5. Behandlungsvorrichtung nach Anspruch 4, wobei die Nadelerfassungseinheit (120) folgendes aufweist:
ein Erkennungselement (122), das außerhalb der Nadel (30) angeordnet ist; und
einen Erkennungssensor (124) zum Antreiben der Nadelerfassungseinheit (40, 70, 80) durch Erkennen des Erkennungselements (122).

6. Behandlungsvorrichtung nach Anspruch 5, wobei das Erkennungselement (120) bandförmig ist.

7. Behandlungsvorrichtung nach Anspruch 5, wobei ein Abstand zwischen dem Erkennungssensor (124) und der Kamera (42) gleich einem Abstand zwischen dem Erkennungselement (122) und der ersten Anzeige (44) ist.

## Revendications

1. Appareil de traitement comportant :
une sonde (10) pour acquérir une image d'une cible en utilisant des ondes ultrasoniques ;
un guide d'aiguille (20) fourni dans la sonde (10); et
une aiguille (30) mobile le long du guide d'aiguille (20) ;
**caractérisé en ce que** l'appareil de traitement comprend en outre :
une unité de mesure (40, 70, 80, 90, 100) pour détecter une distance de déplacement de l'aiguille (30) sans avoir un contact mécanique avec l'aiguille (30) ; et
un contrôleur (50) pour calculer la distance du déplacement de l'aiguille (30) sur la base de la valeur mesurée par l'unité de mesure (40, 70, 80, 90, 100),
dans lequel l'unité de mesure (40) comporte :
un premier indicateur (44) disposé à l'extérieur de l'aiguille (30) pour fournir des informations de longueur; et
une caméra (42) pour capter le premier indicateur (44) et pour transmettre le premier indicateur (44) au contrôleur (50) comme un signal image.

2. Appareil de traitement selon la revendication 1, dans lequel le premier indicateur (44) a une couleur ou modèle variant selon les informations de longueur de l'aiguille (30).

3. Appareil de traitement selon la revendication 1, dans lequel le contrôleur (50) est disposé sur n'importe lequel des éléments comprenant la sonde (10), le guide d'aiguille (20) et une unité principale (60).

4. Appareil de traitement selon la revendication 1, comprenant en outre :
une unité de détection d'une aiguille (120) pour déterminer si l'aiguille a atteint une position de référence,
dans lequel l'unité de mesure (40, 70, 80, 90, 100) est entraînée sur la base d'une détermination de l'unité de détection d'une aiguille (120).

5. Appareil de traitement selon la revendication 4, dans lequel l'unité de détection d'une aiguille (120) comporte :
un organe de reconnaissance (122) disposé à l'extérieur de l'aiguille (30) ; et
un capteur de reconnaissance (124) pour diriger l'unité de détection de l'aiguille (40, 70, 80) en détectant l'organe de reconnaissance (122).

6. Appareil de traitement selon la revendication 5, dans lequel l'organe de reconnaissance (120) est fixé au moyen d'une bande.

7. Appareil de traitement selon la revendication 5, dans lequel une distance entre le capteur de reconnaissance (124) et la caméra (42) est égale à la distance entre l'organe de reconnaissance (122) et le premier indicateur (44).
